(19) **Europäisches Patentamt European Patent Office Office européen des brevets**

(11) **EP 4 714 340 A1**

(12) **EUROPEAN PATENT APPLICATION**

(43) Date of publication:
**25.03.2026 Bulletin 2026/13**

(21) Application number: **24201209.4**

(22) Date of filing: **18.09.2024**

(51) International Patent Classification (IPC):
*A61B 5/022* (2006.01)    *A61B 5/0225* (2006.01)
*A61B 8/04* (2006.01)    *A61B 5/02* (2006.01)
*A61B 5/021* (2006.01)    *A61B 5/00* (2006.01)

(52) Cooperative Patent Classification (CPC):
**A61B 5/0225; A61B 8/04;** A61B 5/02007;
A61B 5/02116; A61B 5/02133; A61B 5/6824;
A61B 5/6843; A61B 8/06; A61B 8/0891;
A61B 2560/0223

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Nederlandse Organisatie voor toegepast- natuurwetenschappelijk onderzoek TNO 2595 DA 's-Gravenhage (NL)**

(72) Inventors:
• **PETERS, Laurens Christiaan Johannes**
  **2595 DA 's-Gravenhage (NL)**
• **VAN NEER, Paul Louis Maria Joseph**
  **2595 DA 's-Gravenhage (NL)**
• **PEETERS, Bart**
  **2595 DA 's-Gravenhage (NL)**
• **VAN HECK, Gerardus Titus**
  **2595 DA 's-Gravenhage (NL)**
• **MATHEW, Denny**
  **2595 DA 's-Gravenhage (NL)**
• **WEBER, Jan**
  **2595 DA 's-Gravenhage (NL)**

(74) Representative: **V.O.
P.O. Box 87930
2508 DH Den Haag (NL)**

(54) **WEARABLE BLOOD PRESSURE MONITORING DEVICE**

(57)    A wearable device (10) comprises a vessel sensor (11) configured to perform measurements (Td,Tp) on a blood vessel (V) for determining a size (D) of the blood vessel (V). A pressure device (12) is configured to controllably apply an external pressure (Px) on a body part for affecting the size (D) of the blood vessel (V). An attachment means (13) is configured to hold the vessel sensor (11) and the pressure device (12) against a respective skin surface (S) of the body part so that the size (D) of the blood vessel (V), as measured by the vessel sensor (11), is affected by the external pressure (Px) applied by the pressure device (12) on the body part. A processor (14) is configured to process the measurements (Td,Tp) as affected by the external pressure (Px).

FIG 1B

EP 4 714 340 A1

**Description**

TECHNICAL FIELD AND BACKGROUND

The present disclosure relates to a wearable device for measuring and/or monitoring physiological parameters, in particular for monitoring blood pressure.

One of the most common methods for blood pressure monitoring involves the use of a pneumatic cuff, which is wrapped around the upper arm and inflates to apply a force in a radial direction. While this method is widely used and clinically validated, it has several drawbacks. The cuff system works rather slow as it applies an uniform pressure around the entire arm circumference, which occludes the artery and temporarily halts blood flow. This intermittent squeezing can be painful and uncomfortable, particularly for obese or elderly patients with fragile skin, and can disrupt sleep when used during night.

Zakrzewski (Zakrzewski, Aaron, Michael. "Arterial blood pressure estimation using ultrasound." PhD diss., Massachusetts Institute of Technology, 2017) offers an alternative approach by using a hand-held ultrasound probe to compress a superficial artery, while simultaneously measuring its cross-sectional area. The method leverages a finite element biomechanical model that incorporates arterial stiffness, wall thickness, and other factors to estimate blood pressure. The requirement for a commercial linear array transducer, which is bulky and power-intensive, makes the system expensive and dedicated to medical professionals only.

While both the upper arm cuff method and the Zakrzewski method can provide incidental blood pressure readings at discrete intervals, they are not suitable for long-term continuous monitoring of blood pressure, which plays an important role in managing various cardiovascular conditions and in the overall assessment of a patient's health.

A less obtrusive blood pressure monitoring method may utilize a finger cuff system to maintain a constant blood volume in a finger through a regulation of applied circumferential pressure. However, the placement of the device on the finger is problematic due to the location's distance from the heart, which can introduce significant measurement errors. Furthermore, the device's positioning on the fingertip is inconvenient for 24-hour monitoring, as it interferes with everyday activities involving the hands and fingers. Additionally, the need for frequent external calibration to ensure accuracy further complicates its use in real-world, continuous monitoring applications.

Given the limitations of current blood pressure monitoring technologies, there is a clear need for a novel solution that is reliable, unobtrusive, comfortable, and capable of providing continuous (24+ hours) and accurate monitoring of absolute blood pressure.

SUMMARY

Aspects of the present disclosure relate to a wearable device comprising a vessel sensor configured to perform measurements on a blood vessel for determining a size of the blood vessel, a pressure device configured to controllably apply an external pressure on a body part for affecting the size of the blood vessel, an attachment means configured to hold the vessel sensor and the pressure device against a respective skin surface of the body part so that the size of the blood vessel, as measured by the vessel sensor, is affected by the external pressure applied by the pressure device on the body part, and a processor configured to process the measurements as affected by the external pressure.

By measuring the size of the blood vessel under a known external pressure, mechanical properties of the blood vessel can be derived, e.g. rigidity, (visco)-elasticity and/or stiffness of the vessel wall, for determining physiological parameters of the blood vessel, e.g. pressure inside the blood vessel. The greater the external pressure applied, the higher the signal-to-noise ratio of the size measurement, but the greater the discomfort to the wearer of the device. The shorter period of applying the pressure is, the more comfortablethe wearable device is to the wearer. The more distinct amounts of external pressure are applied, the more accurately can the physiological parameter be determined.

By processing a set of current measurements of the blood vessel size based on a series of reference measurements, each of the current measurements can be correlated to a blood pressure value, thereby providing an indirect way of measuring the blood pressure. By repeating the series of reference measurements, changes in the elasticity of the blood vessel can be monitored and/or used to calibrate the wearable device. For example, a first series of reference measurements provides indication of a first set of sizes of a blood vessel at respective amounts of external pressure and a second series of reference measurements provides a second set of sizes of the blood vessel at the same respective amounts of external pressure, thereby indicating a change of a physiological parameter, e.g. blood vessel wall elasticity, in the second series of reference measurements as compared to the first series of reference measurements.

By applying a range of respective amounts of external pressure to the skin surface over a period of time, a range of size parameters indicating size of the blood vessel exposed to the respective amounts of external pressure applied via the skin surface can be measured during the period of time, e.g. a cardiac cycle. By measuring the size of the blood vessel continuously and/or for a long-term, physiological parameter, that is dependent on the size, can be determined continuously and/or for a long-term based on (processing of) the size measurements.

Advantageously, a series of different amounts of external pressure can be utilized to calibrate the wearable device for determining an absolute value of blood pressure without the need of using additional calibration devices, e.g. an external cuff pressure device, which may be inconvenient during long-term and/or continuous use. Alternatively, or in addition, relative blood pressure variations may be measured in case the absolute blood pressure value is not required and/or known. Preferably, the calibration cycle is applied initially and after a physiological state change such as taking a medicine or drinking a coffee/alcohol which have direct impact on the vascular system. Alternatively, or in addition, the calibration cycle is applied at predefined time intervals.

By arranging the vessel sensor and pressure device on distinct parts of the skin surface and distributing the external pressure over a surface area larger than the vessel sensor surface, the measurement may feel more comfortable. By using a thin, flexible and/or deformable vessel sensor, external pressure can be applied uniformly and comfortably despite the presence of the vessel sensor. By providing an array of vessel sensors, the size of the blood vessel under the skin surface can be tracked during deformation and/or when the blood vessel gets displaced. Advantageously, the wearable device does not have to be placed accurately on the skin surface when the blood vessel is tracked automatically.

BRIEF DESCRIPTION OF DRAWINGS

These and other features, aspects, and advantages of the apparatus, systems and methods of the present disclosure will become better understood from the following description, appended claims, and accompanying drawing wherein:

> FIGs 1A- 1B illustrate a wearable device attached to a skin surface;
> FIGs 2A-2B illustrate a wearable device attached by a strap;
> FIG 3A illustrates a wearable device applying distinct amounts of pressure to a skin surface;
> FIG 3B shows a relationship between blood pressure and size of a blood vessel;
> FIG 4A shows a relationship between normalized blood pressure and normalized blood vessel diameter;
> FIG 4B shows a relationship between blood pressure and blood vessel diameter for different values of vessel stiffness.

DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

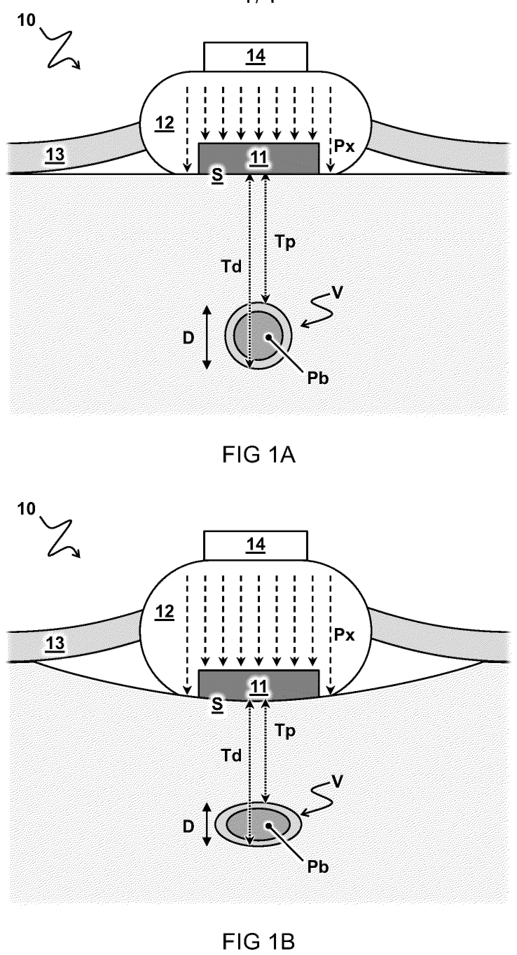

FIGs 1A-1B illustrate a wearable device 10 comprising a vessel sensor 11 configured to perform measurements Td,Tp on a blood vessel V for determining a size D of the blood vessel V. Preferably, the vessel sensor 11 is configured to determine the inner diameter of the blood vessel V. Alternatively, or in addition, the vessel sensor 11 may be configured to determine the outer diameter of the blood vessel V. In some embodiments, measuring the size D of the blood vessel comprises measuring a distance from the vessel sensor 11 to a distal and/or proximal wall of the blood vessel V. Preferably, the blood vessel wall is determined as an interface between the blood vessel V and blood contained therein. Alternatively, or in addition, the blood vessel wall may be determined as an interface between the blood vessel V and surrounding tissue. In another or further embodiment, For example, the distance can be calculated based on a time-of-flight values of ultrasound waves and known speed of ultrasound propagation in the blood vessel V and/or surrounding tissue. Alternatively, or in addition, measuring the size D comprises measuring a shape parameter of the blood vessel V, e.g. deformation of a circular and/or ellipsoidal cross-section of a blood vessel V. Of course, it will understood that measuring a (change in) size D of the blood vessel V may also indicate a (change in) shape of the blood vessel V and/or vice versa. For example, pressing the blood vessel V in one direction may both reduce a size of the blood vessel V in that direction as well as deform the shape of the blood vessel V. In another or further embodiment, measuring the size D comprises measuring optical properties of the blood vessel V and correlating the measured optical properties to a known vessel size and/or reference data.

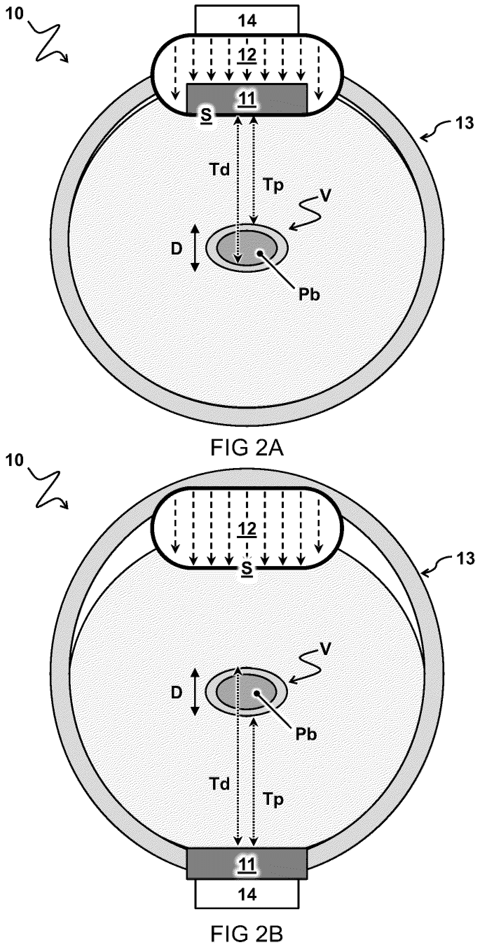

In some embodiments, the wearable device 10 comprises a pressure device 12 configured to apply an external pressure Px to the skin surface S. In one embodiment, the external pressure Px can be applied as a force per unit area or stress acting on the skin surface S. Preferably, the force is applied in a direction towards the skin surface S to press on the skin surface S. Alternatively, or in addition, the force is applied in a direction outwards the skin surface S to pull on the skin surface S. In one embodiment, e.g. as shown in FIGs 1A-1B and 2A, the pressure device 12 comprises a pressure applicator and a pressure/force sensor integrated in a single pressure device 12. In another or further embodiment, e.g. as illustrated in FIG 2B, the pressure applicator and the pressure/force sensor are communicating with each other, but are arranged separately. In yet other or further embodiments, the vessel sensor vessel sensor 11 is configured to facilitate estimating the pressure and/or force applied by a pressure applicator through the skin surface S to the blood vessel V. For example, the vessel sensor 11 may facilitate determining the pressure and/or force on the blood vessel V based on blood vessel depth measurement, and/or composition of the tissue and fat in between the skin surface S and the blood vessel V, or a bone behind the blood vessel V. Alternatively, or in addition, the wearable device 10 comprises an additional sensor configured to improve determining the pressure and/or force on the blood vessel V.

In some embodiments, the pressure applicator comprises a pneumatic system configured to inflate or deflate a set of air bladders or balloons pressing on the skin surface S. In one embodiment, the pneumatic system comprises a pump configured apply positive or negative pressure to the skin surface S. In other or further embodiments, the pressure applicator may comprise hydraulic systems, shape-memory materials, or motorized components. Hydraulic systems use fluid-filled chambers that can be precisely controlled to adjust the pressure, while shape-memory materials respond to temperature or electrical stimuli to change their shape or stiffness, thereby applying pressure in a targeted manner. Motorized systems use small motors or actuators to adjust the pressure automatically, with sensors ensuring that the pressure remains consistent. In yet another or further embodiments, the pressure applicator comprises elastic or tension-controlled straps, which can be manually or automatically adjusted to apply specific levels of external pressure Px to the skin surface S.

In some embodiments, the pressure sensor comprises a piezoresistive sensor, which change its electrical resistance in response to applied pressure. This sensor is highly sensitive and can be integrated into flexible materials, thus suited for wearable applications where conformability to the body and continuous sensing are essential. In other or further embodiments, the pressure sensor comprises a piezoelectric sensor, which generates an electrical charge in response to mechanical stress. This sensor can provide real-time feedback on fast pressure changes, making it suitable for wearable devices which may require quick adjustments, e.g. during an activity. In yet other or further embodiments, the pressure sensor comprises a capacitive sensor, which relies on changes in capacitance between two conductive plates when pressure is applied. This sensor is very sensitive and able to detect very small changes in pressure, making it suitable for applications that require precise pressure measurement. Capacitive sensors are also flexible and can be integrated into soft materials, which is beneficial for wearables that need to conform to the contours of the body without causing discomfort. In other or further embodiments, the pressure sensor comprises MEMS Micro-ElectroMechanical Systems sensor, which are advantageous for their small size, low power consumption, and high accuracy. These sensors can be integrated with other electronic components in a compact form factor, making them ideal for use in smart wearables that need to monitor the external pressure alongside other parameters, e.g. size parameters. In yet other or further embodiments, other sensors for monitoring the external pressure and/or force applied by the pressure device 12 can be envisioned, e.g. optical or strain-based sensors.

In other or further embodiments, the wearable device 10 comprises an attachment means 13 configured to hold the vessel sensor 11 and the pressure device 12 against a skin surface S so that the size D of the blood vessel V, measured by the vessel sensor 11, is affected by the external pressure Px applied by the pressure device 12. In one embodiment, e.g. as shown in FIGs 1A-1B the attachment means 13 comprises adhesive patches, where the wearable device 10 is integrated into a patch that adheres directly to the skin using adhesives. This approach is advantageous for continuous blood pressure monitoring, because it provides close contact with the skin surface and stable attachment, though it may lead to potential skin irritation and limited repositioning. In another or further embodiment, e.g. as shown in FIGs 2A-2B, the attachment means 13 comprises straps, bands, or belts, which wrap around parts of the body like the wrist, chest, or arm. Advantageously, these straps, bands, or belts are adjustable and can function as a pressure applicator when provided with a winding mechanism (not shown) for automatic tightening, e.g. using a motorized winding. In one embodiment, the processor 14 is configured to control the external pressure applied by the strap or band to the skin surface S. In some embodiments, the external pressure Px is applied by the strap, the band, or a belt to the skin surface S indirectly via a cushioning arranged between the attachment means 13 and the skin surface S.

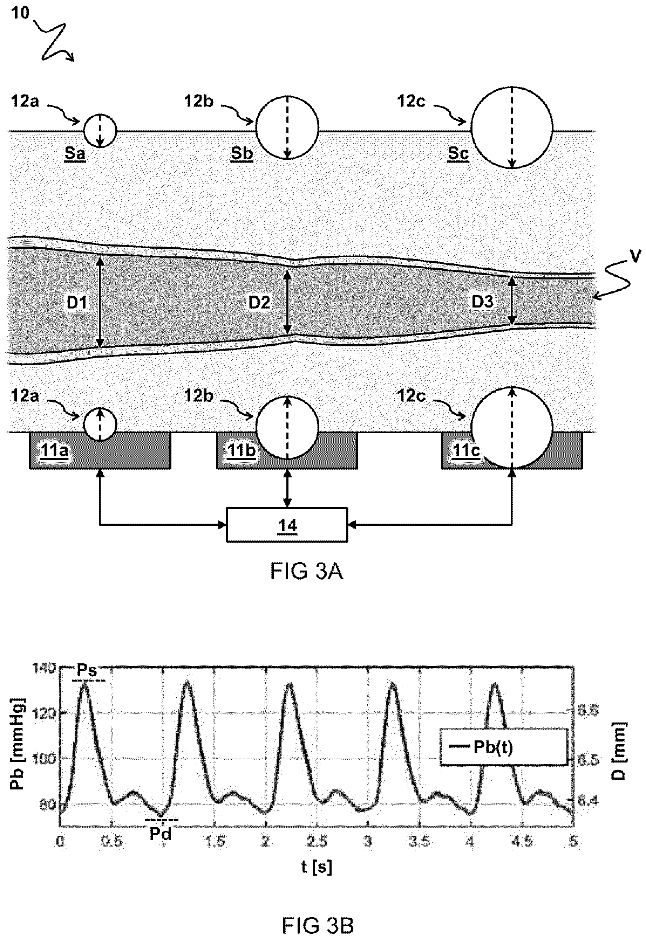

In some embodiments, the wearable device 10 comprises or couples to a processor 14. The processor 14 can be integrated in the wearable device 10 or arranged separately and coupled to the wearable device 10 by an electrical connection or wireless means. In one embodiment, the processor 14 is configured to receive measurements of the size D of blood vessel V from the vessel sensor 11. In another or further embodiment, the processor 14 is configured to process the measurements of the size D or parameters indicating the size D. As described herein, the processing is preferably based on a series of reference measurements which includes respective measurements of the size D at a series of different pressures applied externally. In some embodiments, the reference measurements are performed before (or after) a current measurement of the size D. Accordingly, the reference measurements may be used to recalibrate the wearable device 10. It can also be envisaged that the reference measurements are performed concurrently, e.g. by applying different amounts of external pressure Px at different locations along the length of a blood vessel V and measuring respective size D1,D2,D3 at the different locations, e.g. as shown in FIG 3A. In one embodiment, the same force is applied to areas of different size on the skin surface Sa, Sb, Sc to generate the different amounts of external pressure Px at the different locations. Alternatively, or in addition, areas of the same size are used to apply different amount of force to each of the area on the skin surface S.

In some embodiments, the processor 14 is configured to receive a set of current measurements of the size D from the vessel sensor 11 with a current respective amount of external pressure Px being applied by the pressure device 12. Alternatively, or in addition, the processor 14 is configured to receive, from the vessel sensor 11, a set of current measurements indicating the size D, e.g. voltage, current, shift in optical resonance frequency, et cetera. In other or further embodiments, the processor 14 is further configured to determine a series of reference measurements of the size D as measured by the vessel sensor 11 with different amounts of external pressure Px being applied by the pressure device 12, and process the set of current measurements based on the series of reference measurements. In one embodiment, the set of current measurements comprises a measurement of size D recorded as a function of time at zero external pressure. In another or further embodiment, the series of reference measurements comprises measurements of the size D recorded at non-zero external pressure. In one embodiment, the processor 14 is configured to control the pressure device 12 to apply a respective amount of external pressure Px.

In one embodiment, the external pressure Px applied by the pressure device 12 on the skin surface S to deform the blood vessel V under the skin surface S is actively controlled, e.g. measured and further adjusted. Alternatively, or in addition, a fixed set of different pressures is applied on the skin surface S along a length of the blood vessel V, e.g. as shown in FIG 3A.

In some embodiments, the processor 14 is configured to determine a physiological parameter based on the processing of the size D measurements. Preferably, the physiological parameter comprises blood pressure Pb, e.g. diastolic blood pressure Pd, systolic blood pressure Ps, and or blood pressure determined as a function of time Pb(t). Alternatively, or in addition, other or further physiological parameters can be envisioned, e.g. vessel wall thickness and/or vessel wall elasticity parameter $\alpha$.

FIG 3B shows the relationship between blood pressure Pb inside a blood vessel V and the size D of the blood vessel V as a function of time over a period of five cardiac cycles, during which the blood pressure Pb value changes from its maximum systolic blood pressure value Ps to its minimum diastolic blood pressure value Pd. Clearly, the size D of the blood vessel is the largest at the systolic pressure Ps and the smallest at the diastolic pressure Pd. In some embodiments, determining the physiological parameter comprises measuring the size D of the blood vessel V as a function of time and using a vessel stiffness coefficient $\alpha$ as a parameter to fit a time evolution of the size D(t) of the blood vessel to a reference pressure measurement obtained at a known diastolic pressure Pd inside a blood vessel V of a known diastolic diameter Dd. In other or further embodiments, other fit parameters can be envisaged, e.g. elasticity and/or velocity of a blood pressure wave inside the blood vessel V. In one embodiment, the fit parameter is determined by the vessel sensor 11. In another or further embodiment, the fit parameter is determined by an additional (ultrasound) sensor, e.g. based on pulse wave velocity and/or Doppler measurements, blood vessel wall thickness, stiffness, elasticity, and/or composition measurements.

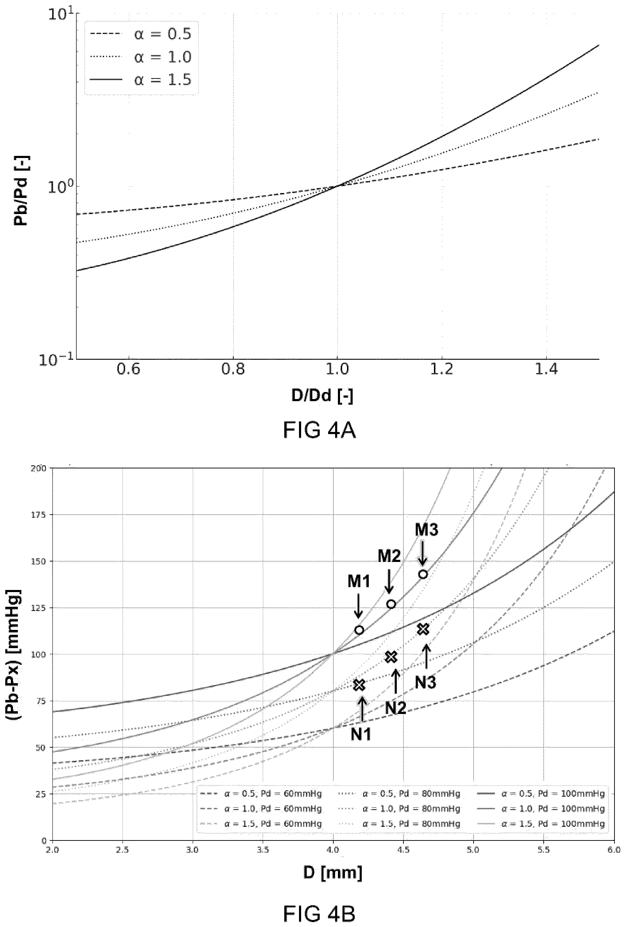

FIGs 4A-4B show a relationship between diameter of a blood vessel and blood pressure inside the blood vessel. The curves are based on an analytical relationship derived by Wang et al. (Wang, Chonghe, et al. "Monitoring of the central blood pressure waveform via a conformal ultrasonic device." Nature biomedical engineering 2.9 (2018): 687-695):

$$P(t) = P_d \cdot e^{\alpha\left(\frac{D^2(t)}{D_d{}^2} - 1\right)}$$

wherein P(t) denotes a blood pressure inside a blood vessel as a function of time, Pd is a diastolic pressure inside the blood vessel, D(t) is a diameter of the blood vessel as a function of time, Dd is a diameter of the blood vessel at the diastolic pressure Pd, and $\alpha$ is an elasticity coefficient of the blood vessel.

FIG 4A shows a relationship between a blood vessel diameter normalized to a diastolic diameter Dd and blood pressure (Pb-Px) normalized to a diastolic blood pressure Pd, with a logarithmic scale on the y-axis. The different line styles in the graph represent varying values of the vessel wall elasticity parameter $\alpha$. The dashed line represents a low vessel wall elasticity parameter α, indicating a vessel of low stiffness. The dotted line represents a medium vessel wall elasticity, indicating a vessel of moderate stiffness. The solid line represents a high vessel wall elasticity, which indicating a vessel of high stiffness. Comparing the vessel wall elasticity parameter values shows that stiffer vessels require more blood pressure Pb for the same increase in size, as indicated by the steeper slope and higher position of the curves above the normalized size value of 1 on the x-axis. Consequently, stiffer vessels require more external pressure Px for the same decrease in size, as indicated by the steeper slope and lower position of the curves below the normalized size value of 1 on the x-axis. This mechanical behavior of blood vessels and vessel stiffness, characterized by the vessel wall elasticity parameter α influences the external pressure needed to achieve specific size changes. The inventors have realized that different amounts of external pressures Px can be utilized to calibrate the wearable device 10 to yield (absolute) blood pressure measurements inside the blood vessel V.

In some embodiments, the processor 14 is configured to apply a calibration cycle, during which the size D is measured at a series of different amounts of external pressure Px, and to determine at least one calibration parameter C based on the calibration cycle. In one embodiment, the at least one calibration parameter C comprises a vessel wall elasticity parameter α, diastolic pressure Pd, and/or diastolic size Dd of the blood vessel V. In another or further embodiment, the processor 14 is configured to determine the physiological parameter based on the calibration parameter C.

In other or further embodiments, determining the calibration parameter C comprises fitting a respective set of measurements M,N of the size D as a function of the respective series of different amounts of external pressure Px to a model or empirical relation between the calibration parameter C and a respective set of different functional relations between the size D and an external pressure Px. In one embodiment, the calibration parameter C may comprise an offset value, e.g. a pressure value, a scaling factor, e.g. a pressure scaling factor, or a function transforming value of the measured physiological parameter to an absolute/calibrated value of the physiological parameter. In some embodiments, the model comprises a set of calibration curves, a look-up-table, a set of analytical expressions, a set of empirical values, simulated data, or a trained neural network relating values of a respective vessel size D, e.g. a diameter, measured at a series of different amounts of external pressure Px for different mechanical properties of the vessel, e.g. elasticity, to a physiological parameter, e.g. absolute diastolic blood pressure. By accounting for the non-linear mechanical properties of the vessel in the calibration cycle, absolute blood pressure can be determined under variable physiological conditions, e.g. change in the vessel wall stiffness as a result of sleeping, taking vasoconstrictors, and/or drinking coffee or alcohol.

In some embodiments, determining the calibration parameter C comprises obtaining a series of measurements M1,M2,M3 of different sizes D1,D2,D3 of a blood vessel V measured at the respective series of different amounts of external pressure Px and fitting the series of different size measurements M1,M2,M3 to a calibration curve relating size D of a blood vessel V to an absolute blood pressure Pb-Px inside the blood vessel V. In other or further embodiments, determining the calibration parameter C comprises obtaining, after a predefined period of time and/or physiological state change, a series of new measurements N1,N2,N3 of different sizes D1,D2,D3 of the blood vessel V measured at the respective series of different amounts of external pressure Px and repeating the fitting, e.g. as illustrated in FIG 4B by the respective series of circles and cross markers, wherein the respective calibration curve is the curve that is the closest to the respective set of markers. In one embodiment, the closest calibration curve may be determined using a least square method. Other mathematical fitting methods can be envisioned too.

In some embodiments, the external pressure Px applied by the pressure device is varied according to a periodic function, e.g. sinusoidal function. Alternatively, or in addition, intermittent, or continuous function can be used too. In one embodiment, the measurements Td, Tp is processed based on the periodic function for increasing a signal-to-noise ratio of the measurement of size D of the blood vessel V as measured by the vessel sensor 11. In one embodiment, the processor 14 triggers the vessel sensor 11 to measure the size D based on the periodic function. In another or further embodiment, the processor 14 is configured to isolate the effect of the applied external pressure Px from motion artifacts based on a band-pass filter matching the periodic function. Preferably, frequency of the periodic variation is chosen above the upper sense tactility frequency of humans, e.g. above 200 Hz. In another or further embodiment, the calibration cycle is applied at an (absolute) external pressure Px that is lower than the lowest (absolute) pressure inside the blood vessel V, thereby deforming the blood vessel V while preventing complete closure of the blood vessel V. For example, the external pressure Px applied during the calibration cycle is lower than the pressure inside the blood vessel V by a factor of two, five, ten, up to one hundred or more. The lower the external pressure Px, the more comfortable the wearable device 10 is to the wearer, but the more difficult it is to measure the size D of the blood vessel V affected by the external pressure Px. Preferably, the external pressure Px is applied in a range 1 - 50 mmHg (0.1 - 7 kPa), more preferably in a range 1-30 mmHg (0.1 - 4 kPa). Alternatively, or in addition the external pressure Px can be applied at a lower range in case of sufficient signal-to-noise ratio of the size D measurements, e.g. in case of a slim/lean wearer.

In one embodiment, the external pressure is applied by a simple pressure device capable of delivering the maximum external pressure of 100 mmHg, 50 mmHg or 30 mmHg. The lower the maximum external pressure, the simpler and the more energy efficient the pressure device can be. Alternatively, or in addition, the external pressure can be applied by a more sophisticated pressure device capable of delivering higher external pressure, the pressure device configured to limit the external pressure to the maximum of 100 mmHg, 50 mmHg or 30 mmHg.

In other or further embodiments, the external pressure Px is applied for a fixed period of time. The longer the external pressure Px is applied, the more measurements of the size D of the blood vessel V at the respective external pressure Px can be obtained. Preferably, the external pressure Px is applied for a period of 0, 1 - 20 seconds. Alternatively, or in addition, the external pressure Px can be applied for a longer period of time, e.g. up to 60 or more seconds.

In yet other or further embodiments, the external pressure Px is applied in a ramp up/down fashion. The longer the ramp up/down, the more comfortable is the wearable device 10 to the wearer. Preferably, the ramp up/down takes 1 - 60 seconds. Even more preferably, the maximum external pressure variation applied is less than 50 mmHg per second, 10 mmHg per second, or 0.1 mmHg per second. The lower the external pressure variation, the more comfortable the wearable device 10 is, e.g. during a long-term or continuous monitoring.

In other or further embodiments, the external pressure Px is hold constant for a fixed period of time after it has ramped up to the maximum value and/or before it starts ramping down to the minimum value.

In some embodiments, surface of the pressure device 12 that is in contact with the skin surface S is larger than surface of the vessel sensor 11, e.g. factor of 1.5 to 5 larger. In other or further embodiments, the vessel sensor 11 is arranged on a first part of the skin surface and the pressure device 12 is arranged on a second part of the skin surface. In one embodiment, e.g. as shown in FIG 2A, the first part overlaps at least partially with the second part. In another or further embodiment, e.g. as shown in FIG 2B, the first part is separate from the second part. In other or further embodiments, the pressure device 12 comprises one or more surfaces contacting the skin surface of the body part at one or more locations Sa,Sb,Sc. In one embodiment, the one or more surfaces form a part of the attachment means 13.

In some embodiments, measuring the series comprises measuring the respective size D at the minimum of three different values of the external pressure Px. The more values of different external pressure Px are used, the better the measurement can be correlated to a respective predetermined relation between the size D and the physiological parameter such as the blood pressure Pb. Preferably at least at four, five, six, ten or more different values of the external pressure Px are used. The number of measurement points needed to fit a measurement to a model or a an empirical relation depends on a several factors, including the desired accuracy, the complexity of the model, and the variability of the data. Generally, more measurement points lead to a more accurate and reliable fit. For a simple model with one or two parameters, a minimum of three to five points might be sufficient to get a rough fit, especially if the data follows a clear trend. However, to capture the full behavior of the relationship and account for any non-linearities, it's advisable to have at least 10 to 15 points spread across the range of interest. If the model is more complex or if there is significant noise in the data, additional points are needed to ensure that the fit is robust and not overly influenced by outliers or random variations. In practice, it's also important to ensure that the points are well-distributed across the range of the size parameter values to avoid biasing the fit toward certain regions.

In other or further embodiments, measuring the series comprises measuring a series of sizes D1,D2,D3 at different values of the external pressure Px. For example, the series of reference measurements comprises at least two, preferably three, up to five or more measurements. In another or further embodiment, each size parameter in the series of the sizes D1,D2,D3 is measured at a specific cardiac cycle phase. In one embodiment, the series of size parameters are all measured for the systolic phase. For example, this may be determined by using the maximum measured size Dmax in each period. In another or further embodiment, the series of size parameters are all measured for the diastolic phase. For example, this may be determined by using the minimum measured size Dmin in each period. It can also be envisage to use other or further points of the cardiac cycle phase. For example, the specific point in the cardiac cycle phase may be derived from a graph of the diameter as function of time and/or using external synchronization, e.g. using ECG gating.

In other or further embodiment, the series of size parameters are all measured at the same time at distinct spatial locations along the blood vessel V. For example, each spatial location can be exposed to a different pressure such as to measure the series of size parameters at different values of the external pressure Px. In yet other or further embodiments, the series of size parameters are measured at different amounts of external pressure Px at different times at distinct spatial locations along the blood vessel V. For example, a first external pressure Pxa is applied at a first frequency Fa at a first skin surface Sa, second external pressure Pxb is applied at a second frequency Fb at a second skin surface Sb, and a third external pressure Pxb is applied at a third frequency Fb at a third skin surface Sc. In one embodiment, the respective frequencies differ by at least half a hertz, preferably, one hertz, up to five hertz or more.

In some embodiments, the vessel sensor 11 comprises means for tracking the blood vessel V under the skin surface S. In one embodiment, the means for tracking the blood vessel V comprises an ultrasound transducer configured to detect flow of blood through the blood vessel V, e.g. using a Doppler effect. By analyzing the change in frequency of the ultrasound waves reflecting form moving blood cells inside the blood vessel V, information about blood flow and vessel location can be obtained. In another or further embodiment, the means for tracking the blood vessel V comprises an optical sensor. For example, a near-infrared sensor emits light that penetrates the skin surface S and that is absorbed differently by blood than by the surrounding tissue, thereby providing contrast between the blood vessel V and the surrounding tissue for tracking the blood vessel V under the skin surface S. In another example, an infrared sensor detects heat emitted by a blood vessel V under the skin surface S, thereby providing contrast between the blood vessel V and the surrounding colder tissue for tracking the blood vessel V under the skin surface S.

In some embodiments, the vessel sensor 11 comprises at least one ultrasound transducer configured to measure the size D of the blood vessel V based on coupling ultrasound energy through the skin surface S to the blood vessel V and receiving, as a function of time, portion of the ultrasound energy reflected from a proximal and distal wall of the blood vessel V back to the vessel sensor 11 to estimate time-of-flight values of the ultrasound waves travelling from the vessel sensor 11 through the skin surface S to the respective wall of the blood vessel V and back. In one embodiment, the ultrasound transducer is a thin film flexible transducer configured to deform following a curvature of the skin surface S for uniformly applying the external pressure Px through the ultrasound transducer on the body part. In other or further embodiments (not shown), the vessel sensor 11 comprises an optical sensor, e.g. an infrared sensor, configured to monitor optical properties of the blood, e.g. absorption or scattering of hemoglobin, contained in the blood vessel V and varying as a function of the diameter of the blood vessel V.

In one embodiment, the ultrasound device comprises an array of ultrasound elements coupled to the processor 14, wherein the processor 14 is configured to select the ultrasound element with highest amplitude of ultrasound signal reflected from the blood vessel V. In one embodiment, the vessel sensor 11 comprises an array of ultrasound elements arranged at a distance from each other and configured to measure the size D of the blood vessel V. In one embodiment, the processor 14 is configured to evaluate the alignment of each of the ultrasound elements with respect to a center of a blood vessel V and further process measurements from the respective ultrasound element that is the most aligned with the center of the blood vessel V. Preferably, the alignment to the center of a blood vessel V is based on signal amplitude of the ultrasound signal reflected from the blood vessel V. Alternatively, or in addition, other parameters or methods can be envisioned to select an ultrasound element from the array that provides the best measurement of the side D of the blood vessel V, e.g. distance to the blood vessel V, signal to noise ratio of the measurement, amount of measured change in size D of the blood vessel following applying the external pressure Px, cross-correlation between ultrasound signals measured over a period of time, et cetera.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

**Claims**

1. A wearable device (10) comprising

   a vessel sensor (11) configured to perform measurements (Td,Tp) on a blood vessel (V) for determining a size (D) of the blood vessel (V);
   a pressure device (12) configured to controllably apply an external pressure (Px) on a body part for affecting the size (D) of the blood vessel (V);
   an attachment means (13) configured to hold the vessel sensor (11) and the pressure device (12) against a respective skin surface (S) of the body part so that the size (D) of the blood vessel (V), as measured by the vessel sensor (11), is affected by the external pressure (Px) applied by the pressure device (12) on the body part;
   a processor (14) configured to process the measurements (Td,Tp) as affected by the external pressure (Px).

2. The wearable device (10) according to the preceding claim, wherein the processor (14) is configured to

   receive a set of current measurements of the size (D) from the vessel sensor (11) with a current respective amount of external pressure (Px) being applied by the pressure device (12), determine a series of reference measurements of the size (D) as measured by the vessel sensor (11) with different amounts of external pressure (Px) being applied by the pressure device (12),

and

process the set of current measurements based on the series of reference measurements.

3. The wearable device (10) according to the preceding claim, wherein the series of reference measurements comprise measurements of the size (D) of a blood vessel (V) by the vessel sensor (11) with at least three different amounts of external pressure (Px) applied by the pressure device (12) sequentially at the same location and/or simultaneously at distinct locations along the blood vessel (V).

4. The wearable device (10) according any of the two preceding claims, wherein measuring the series of reference measurements comprises measuring a series of sizes (D1,D2,D3) at different values of the external pressure (Px), wherein each size parameter in the series of the sizes (D1,D2,D3) is measured at a specific cardiac cycle phase.

5. The wearable device (10) according to any of the preceding claims, wherein the processor (14) is configured to control the pressure device (12) to apply a respective amount of external pressure (Px) and/or to receive an additional sensor data for determining the pressure and/or force applied on the blood vessel (V) by the pressure device (12).

6. The wearable device (10) according to any of the preceding claims, wherein the pressure device (12) is configured to exclusively apply external pressures (Px) in a range below 100 mmHg.

7. The wearable device (10) according to any of the preceding claims, wherein the pressure device (12) is configured to vary the external pressure (Px) with a maximum external pressure variation of less than 50 mmHg per second.

8. The wearable device (10) according to any of the preceding claims, wherein the pressure device (12) is controlled to vary the external pressure (Px) according to a periodic function, and the processor (14) is configured to process the measurements (Td,Tp) based on the periodic function.

9. The wearable device (10) according to any of the preceding claims, wherein the processor is configured to

apply a calibration cycle during which a series of reference measurements of a size (D) is measured at a series of different amounts of applied external pressure (Px); and
determine at least one calibration parameter (C) based, at least in part, on the calibration cycle; wherein the calibration parameter (C) is determined based, at least in part, on fitting the measured reference measurements of the size (D) as a function of the series of different amounts of applied external pressure (Px) to a model or empirical relation between the calibration parameter (C) and a respective set of different functional relations between the size (D) and external pressure (Px).

10. The wearable device (10) according to the preceding claim, wherein the processor (14) is configured to determine a physiological parameter such as a current blood pressure (Pb) based on the processing of the measurements of the size (D) in combination with at least one calibration parameter (C).

11. The wearable device (10) according to any of the preceding claims, wherein, when the wearable device (10) is worn on the body part by the attachment means (13), a surface area of the pressure device (12) pressing onto the skin surface (S) of the body part is larger than a surface area of the vessel sensor (11) by at least a factor one and a half.

12. The wearable device (10) according to any of the preceding claims, wherein the vessel sensor (11) comprises at least one ultrasound transducer configured to measure the size (D) of the blood vessel (V) based on the coupling of ultrasound wave energy through the skin surface (S) to the blood vessel (V) and receiving, as a function of time, a portion of the ultrasound wave energy reflected from a proximal and distal wall of the blood vessel (V) back to the vessel sensor (11) for measuring at least one of the time-of-flight, phase, and frequency value of the respective ultrasound waves travelling from the vessel sensor (11) through the skin surface (S) to the respective wall of the blood vessel (V) and back to the vessel sensor (11).

13. The wearable device (10) according to the preceding claim, wherein the ultrasound transducer is a thin film flexible transducer configured to deform following a curvature of the skin surface (S) for uniformly applying the external pressure (Px) through the ultrasound transducer on the body part.

14. The wearable device (10) according to any of the preceding claims, wherein the vessel sensor (11) comprises means for tracking the blood vessel (V) under the skin surface (S).

15. The wearable device (10) according to the three preceding claims, wherein the ultrasound transducer comprises an array of ultrasound elements coupled to a processor (14), wherein the processor (14) is configured to evaluate alignment of each of the ultrasound elements with respect to a center of a blood vessel (V) and select the ultrasound element that is the most aligned with the center of the blood vessel (V).

FIG 1A

FIG 1B

FIG 2A

FIG 2B

FIG 3A

FIG 3B

FIG 4A

FIG 4B

## EUROPEAN SEARCH REPORT

**Application Number**

EP 24 20 1209

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/153755 A1 (SRINIVASAN MANDAYAM A [US] ET AL) 27 May 2021 (2021-05-27) | 1-14 | INV.<br>A61B5/022 |
| Y | * paragraphs [0034] - [0055], [0058] - [0068]; claims 1,8,11,18-20 * | 15 | A61B5/0225<br>A61B8/04 |
| | ----- | | |
| X | US 2013/158418 A1 (MIZUKAMI HIROMITSU [JP]) 20 June 2013 (2013-06-20)<br>* claim 1; figures 1-4,6,7,9-11 * | 1,4,5 | ADD.<br>A61B5/02<br>A61B5/021<br>A61B5/00 |
| | ----- | | |
| X | US 2016/038117 A1 (TAMADA NATSUMI [JP]) 11 February 2016 (2016-02-11)<br>* claim 1 * | 1 | |
| | ----- | | |
| Y | US 2016/095572 A1 (AGUREN DERRICK [US]) 7 April 2016 (2016-04-07)<br>* the whole document * | 15 | |
| | ----- | | |

**TECHNICAL FIELDS SEARCHED (IPC)**

A61B

The present search report has been drawn up for all claims

| Place of search | Date of completion of the search | Examiner |
|---|---|---|
| Berlin | 12 February 2025 | Kronberger, Raphael |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 20 1209

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2025

| Patent document cited in search report | Publication date | Patent family member(s) | | Publication date |
|---|---|---|---|---|
| US 2021153755 A1 | 27-05-2021 | AU | 2019249818 A1 | 15-10-2020 |
| | | BR | 112020019909 A2 | 05-01-2021 |
| | | CA | 3094403 A1 | 10-10-2019 |
| | | CN | 112512414 A | 16-03-2021 |
| | | CN | 118902421 A | 08-11-2024 |
| | | EP | 3773183 A1 | 17-02-2021 |
| | | EP | 4166074 A1 | 19-04-2023 |
| | | EP | 4434472 A2 | 25-09-2024 |
| | | IL | 277022 A | 29-10-2020 |
| | | JP | 7422672 B2 | 26-01-2024 |
| | | JP | 2021518179 A | 02-08-2021 |
| | | JP | 2024038392 A | 19-03-2024 |
| | | KR | 20210019404 A | 22-02-2021 |
| | | KR | 20240151249 A | 17-10-2024 |
| | | RU | 2020131254 A | 05-05-2022 |
| | | SA | 520420190 B1 | 13-05-2024 |
| | | SG | 11202008316U A | 29-09-2020 |
| | | US | 2021153755 A1 | 27-05-2021 |
| | | US | 2024099597 A1 | 28-03-2024 |
| | | US | 2024245312 A1 | 25-07-2024 |
| | | WO | 2019195120 A1 | 10-10-2019 |
| | | ZA | 202005840 B | 22-02-2023 |
| US 2013158418 A1 | 20-06-2013 | CN | 103156588 A | 19-06-2013 |
| | | JP | 6098101 B2 | 22-03-2017 |
| | | JP | 2013144098 A | 25-07-2013 |
| | | US | 2013158418 A1 | 20-06-2013 |
| US 2016038117 A1 | 11-02-2016 | CN | 105361906 A | 02-03-2016 |
| | | JP | 2016036644 A | 22-03-2016 |
| | | US | 2016038117 A1 | 11-02-2016 |
| US 2016095572 A1 | 07-04-2016 | US | 2016095572 A1 | 07-04-2016 |
| | | US | 2018360412 A1 | 20-12-2018 |
| | | WO | 2016057233 A1 | 14-04-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Arterial blood pressure estimation using ultrasound. **ZAKRZEWSKI, AARON, MICHAEL**. PhD diss.. Massachusetts Institute of Technology, 2017

- **WANG, CHONGHE et al.** Monitoring of the central blood pressure waveform via a conformal ultrasonic device. *Nature biomedical engineering*, 2018, vol. 2 (9), 687-695